# EUROPÄISCHE PATENTSCHRIFT

(11) **EP 1 003 730 B1**
(45) Veröffentlichungstag und Bekanntmachung des Hinweises auf die Patenterteilung: **30.05.2007**
(21) Anmeldenummer: 98943774.4
(22) Anmeldetag: 23.07.1998
(51) Int. Cl.: C07D 261/04, C07D 307/58, A01N 43/80, A01N 43/08

(54) **2-BENZOYL-CYCLOHEXAN-1,3-DIONE**
2-BENZOYL-CYCLOHEXANE-1,3-DIONES
2-BENZOYLE-CYCLOHEXANE-1,3-DIONES

(30) Priorität: 07.08.1997 DE 19734201
(43) Veröffentlichungstag der Anmeldung: 31.05.2000
(73) Patentinhaber: BASF Aktiengesellschaft, 67056 Ludwigshafen (DE)
(72) Erfinder: ENGEL, Stefan, D-55286 Wörrstadt (DE); RHEINHEIMER, Joachim, D-67063 Ludwigshafen (DE); BAUMANN, Ernst, D-67373 Dudenhofen (DE); von DEYN, Wolfgang, D-67435 Neustadt (DE); HILL, Regina, Luise, D-67346 Speyer (DE); MAYER, Guido, D-67433 Neustadt (DE); MISSLITZ, Ulf, D-67433 Neustadt (DE); WAGNER, Oliver, D-67061 Ludwigshafen (DE); WITSCHEL, Matthias, D-67061 Ludwigshafen (DE); OTTEN, Martina, D-67069 Ludwigshafen (DE); WALTER, Helmut, D-67283 Obrigheim (DE); WESTPHALEN, Karl-Otto, D-67346 Speyer (DE)
(86) Internationale Anmeldenummer: PCT/EP1998/004636
(87) Internationale Veröffentlichungsnummer: WO 1999/007688

(56) Entgegenhaltungen:
- EP-A- 0 186 118
- EP-A- 0 186 119
- EP-A- 0 186 120
- EP-A- 0 278 742
- EP-A- 0 317 158
- EP-A- 0 320 864
- WO-A-91/00260
- WO-A-96/26200
- US-A- 4 780 127
- US-A- 4 954 165
- PATENT ABSTRACTS OF JAPAN vol. 96, no. 5, 31. Mai 1996 & JP 08 020554 A (HOKKO CHEM IND CO LTD), 23. Januar 1996

## Beschreibung

Die vorliegende Erfindung betrifft substituierte 2-Benzoyl-cyclohexan-1,3-dione der Formel I 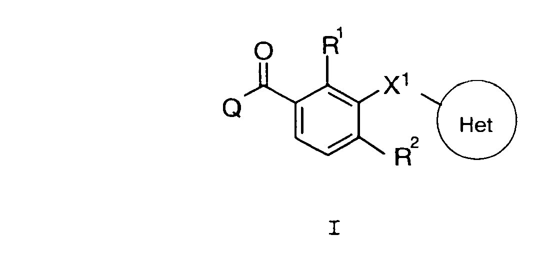 in der die Substituenten folgende Bedeutung haben:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR³ oder -S(O)ₙR³;
- R²: Wasserstoff oder ein wie voranstehend unter R¹ genannter Rest;
- R³: Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/ oder eine bis drei der folgenden Gruppen tragen können:
Hydroxy, Mercapto, Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-slkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits durch ein bis drei Halogenatome und/oder einen oder zwei Reste aus folgender Gruppe: Nitro, Cyano, Methyl, Trifluormethyl, Methoxy, Trifluormethoxy oder Methoxycarbonyl substituiert sein
können;

- n: 0, 1 oder 2;
- Q: ein in 2-Stellung verknüpfter Cyclohexan-1,3-dion-Ring der Formel II 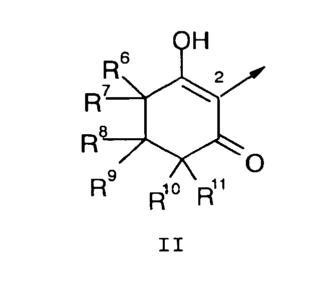
wobei R⁶, R⁷, R⁹ und R¹¹ für Wasserstoff oder C₁-C₄-Alkyl stehen;
- R⁸: für Wasserstoff, C₁₋₄-Alkyl oder C₃-C₄-Cycloalkyl steht, wobei die beiden letztgenannten Gruppen einen bis drei der folgenden Substituenten tragen können: Halogen, C₁-C₄-Alkylthio oder C₁-C₄-Alkoxy;
oder
für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch einen bis drei C₁-C₄-Alkylreste substituiert sein können;
- R¹⁰: für Wasserstoff, C₁-C₄-Alkyl oder C₁-C₆-Alkoxycarbonyl steht;
oder
- R⁸ und R¹¹: gemeinsam eine π-Bindung oder einen drei- bis sechsgliedrigen carbocyclischen Ring bilden;
oder
die CR⁸R⁹-Einheit durch C=O ersetzt sein kann.
- X¹: eine C₁-C₂-Alkylen- oder eine C₂-Alkinylenkette;
- R⁴: Wasserstoff, C₁-C₆-Alkyl, C₂-C₆-Alkenyl, C₂-C₆-Alkinyl, Phenyl, Phenyl-C₁-C₆-alkyl; wobei die genannten Alkyl-, Alkenyl oder Alkinylreste partiell oder vollständig halogeniert sein können und/ oder durch einen oder mehrere der folgenden Reste substituiert sein können:
Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
- Het: eine drei- bis sechsgliedrige, teilweise oder vollständig gesättigte, heterocyclische Gruppe mit bis zu drei Heteroatomen ausgewählt aus der Gruppe:
Stickstoff, Sauerstoff oder Schwefel,
wobei die genannte heterocyclische Gruppe partiell oder vollständig halogeniert sein kann und/oder durch R⁵ substituiert sein kann;
- R⁵: Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
sowie deren landwirtschaftlich brauchbaren Salze.

Außerdem betrifft die Erfindung Verfahren und zwischenprodukte zur Herstellung von Verbindungen der Formel I, Mittel welche diese enthalten, sowie die Verwendung der Verbindungen der Formel I und diese enthaltende Mittel zur Schadpflanzenbekämpfung.

Aus der Literatur, beispielsweise aus JP 08/020554, EP-A 278 742, EP-A 298 680, EP-A 320 864 und WO 96/14285 sind 2-Benzoylcyclohexan-1,3-dione bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die erfindungsgemäßen 2-Benzoyl-cyclohexan-1,3-dione der Formel I sowie deren herbizide Wirkung gefunden.

Ferner wurden herbizide Mittel gefunden, die die Verbindungen I enthalten und eine sehr gute herbizide Wirkung besitzen. Darüber hinaus wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon erfaßt.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die Variable Q stellt einen in 2-Stellung verknüpften Cyclohexan-1,3-dionring der Formel II dar, 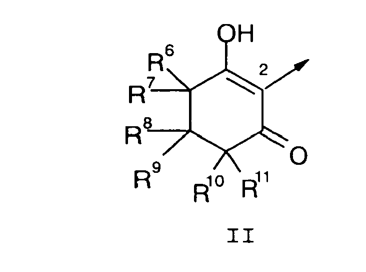 wobei II auch stellvertretend für die tautomeren Formeln II' und II" steht, 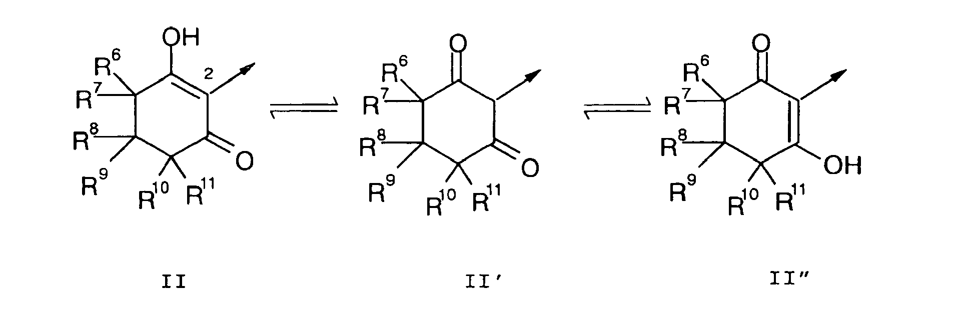 wobei R⁶ bis R¹¹ die zuvor genannten Bedeutungen aufweisen.

### Verfahren A:

Umsetzungen von Cyclohexan-1,3-dion der Formel II mit einer aktivierten Carbonsäure IIIa oder einer Carbonsäure IIIb die vorzugsweise in situ aktiviert wird, zu dem Acylierungsprodukt IV und anschließende Umlagerung zu den erfindungsgemäßen Verbindungen der Formel I.

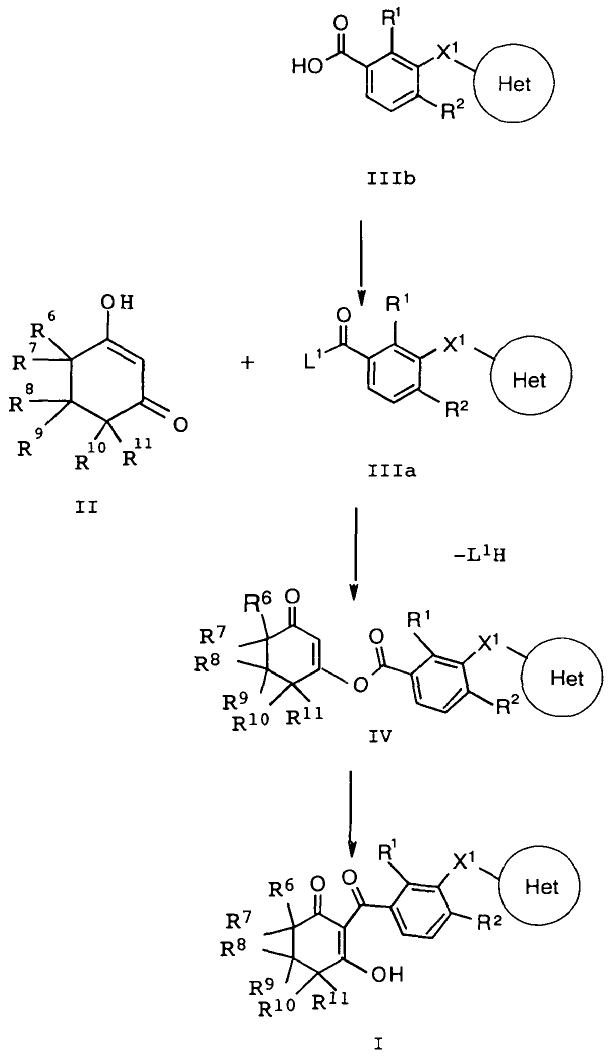

L¹ steht für eine nucleophil austauschbare Abgangsgruppe, wie Halogen z. B. Brom, Chlor, Hetaryl, z. B. Imidazolyl, Pyridyl, Carboxylat, z. B. Acetat, Trifluoracetat etc.

Die aktivierte Carbonsäure kann direkt eingesetzt werden, wie im Fall der Carbonsäurehalogenide oder in situ erzeugt werden, z. B. mit Dicyclohexylcarbodiimid, Triphenylphosphin/Azodicarbonsäureester, 2-Pyridindisulfit/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z. B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z. B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0 - 10 °C abzukühlen. Anschließend rührt man bei 20 - 100 °C, vorzugsweise bei 25 - 50 °C, bis die Umsetzung vollständig ist. Die Aufarbeitung erfolgt in üblicher Weise, z. B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel IV vorzugsweise durch Chromatographie gereinigt. Es ist aber auch möglich, den rohen Enolester der Formel IV ohne weitere Reinigung zur Umlagerung einzusetzen.

Die Umlagerung der Enolester der Formel IV zu den Verbindungen der Formel I erfolgt zweckmäßigerweise bei Temperaturen von 20 bis 40 °C in einem Lösungsmittel und in Gegenwart einer Base sowie gegebenenfalls in Gegenwart einer Cyanoverbindung.

Als Lösungsmittel können z. B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z. B. in einer Menge von 5 bis 15, vorzugsweise 10 Molprozent, bezogen auf den Ester, eingesetzt.

Besonders bevorzugt werden Alkalicarbonate, wie Kaliumcarbonat, in Acetonitril oder Dioxan eingesetzt.

Die Aufarbeitung kann in an sich bekannter Weise erfolgen. Das Reaktionsgemisch wird z. B. mit verdünnter Mineralsäure, wie 5 %ige Salzsäure oder Schwefelsäure, angesäuert, mit einem organischen Lösungsmittel, z. B. Methylenchlorid, Essigsäureethylester extrahiert. Der organische Extrakt kann mit 5 - 10 %iger Alkalicarbonatlösung, z. B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

(Beispiele für die Darstellung von Estern von Hydroxypyrazolen und für die Umlagerung der Ester sind z. B. in EP-A 282 944 oder US 4 643 757 genannt).

Die Benzoesäuren der Formel III sind neu, 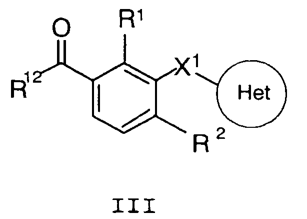 wobei die variablen folgende Bedeutung haben:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR³ oder -S(O)ₙR3;
- R²: Wasserstoff oder ein wie voranstehend unter R¹ genannter Rest;
- R³: Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/ oder eine bis drei der folgenden Gruppen tragen können:
Hydroxy, Mercapto, Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits durch ein bis drei
Halogenatome, und/oder einen oder zwei Reste aus folgender Gruppe: Nitro, Cyano, Methyl, Trifluormethyl, Methoxy, Trifluormethoxy oder Methoxycarbonyl substituiert sein können;
- n: 0, 1 oder 2;
- X¹: eine C₁-C₂-Alkylen- oder eine C₂-Alkinylenkette;
- R⁴: Wasserstoff, C₁-C₆-Alkyl, C₂-C₆-Alkenyl, C₂-C₆-Alkinyl, Phenyl, Phenyl-C₁-C₆-alkyl, wobei die genannten Alkyl-, Alkenyl oder Alkinylreste partiell oder vollständig halogeniert sein können und/ oder durch einen oder mehrere der folgenden Reste substituiert sein können:
Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio,
C₁-C₄-Halogenalkylthio, C₁-C₄-Alkoxy, C₁-C₄-Halogenalkoxy;

- Het: eine drei- bis sechsgliedrige, teilweise oder vollständig gesättigte, heterocyclische Gruppe mit bis zu drei Heteroatomen ausgewählt aus der Gruppe:
Stickstoff, Sauerstoff oder Schwefel,
wobei die genannte heterocyclische Gruppe partiell oder vollständig halogeniert sein kann und/oder durch R⁵ substituiert sein kann;
- R⁵: Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
- R¹²: Hydroxy oder ein hydrolysierbarer Rest.

Beispiele für hydrolysierbare Reste sind Alkoxy-, Phenoxy-, Alkylthio-, Phenylthioreste, die substituiert sein können, Halogenide, Hetarylreste, die über Stickstoff gebunden sind, Amino-, Iminoreste, die substituiert sein können, etc.

Bevorzugt sind Benzoesäurehalogenide IIIa mit L¹ = Halogen (≙ III mit R¹² = Halogen), 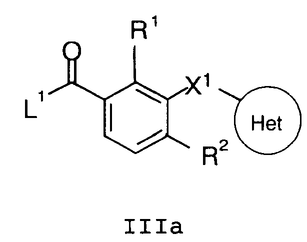 wobei die Variablen R¹, R², X¹ und Het die unter Formel III genannte Bedeutung haben und
- L¹: Halogen, insbesondere Chlor oder Brom, bedeuten.
Ebenso bevorzugt sind Benzoesäuren der Formel IIIb (≙ III mit R¹²= Hydroxy), 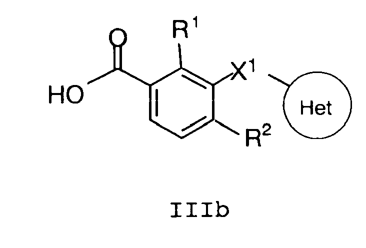 wobei die Variablen R¹, R², X¹ und Het die unter Formel III genannte Bedeutung haben.

Ebenso bevorzugt sind Benzoesäureester der Formel IIIc (≙ III mit R¹² = C₁-C₆-Alkoxy), 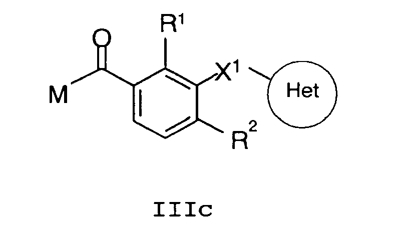 wobei die Variablen R¹, R², X¹ und Het die unter Formel III genannte Bedeutung haben und
- M: C₁-C₆-Alkoxy
bedeutet.

Die Verbindungen der Formel IIIa (mit L¹ = Halogen) können in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel IIIb mit Halogenierungsreagentien wie Thionylchlorid, Thionylbromid, Phosgen, Diphosgen, Triphosgen, Oxalylchlorid, Oxalylbromid dargestellt werden.

Die Benzoesäuren der Formel IIIb können u. a. durch Verseifung der Benzoesäureester der Formel IIIc (mit M = C₁-C₆-Alkoxy) erhalten werden.

Die erfindungsgemäßen Benzoesäureester der Formel IIIc sind nach verschiedenen literaturbekannten Methoden (z. B. a. G. Dittus in Houben-Weyl, Methoden der Organischen Chemie, Band VI/3, Sauerstoff-Verbindungen I, 4. Aufl., S. 493 ff., Georg Thieme Verlag, 1965; b. T. L. Gilchrist, Heterocyclenchemie, 2. Aufl., Verlag Chemie, 1995) darstellbar, wie in den nachfolgenden Beispielen illustriert.

### Verfahren B:

Metallierung geeigneter Benzoesäureester IIIc in *ortho*-Position zur Esterfunktion mit starken, metallorganischen Basen und anschließende 1,2-Addition einer Carbonylverbindung V liefert die erfindungsgemäßen Benzoesäureester IIIe, 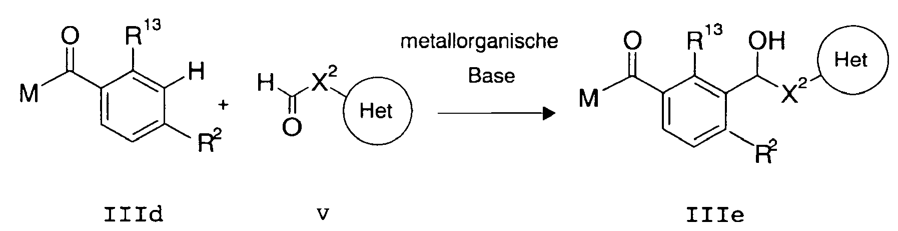 wobei R¹³ einen zur ortho-Metallierung geeigneten Substituenten R¹ darstellt (z.B. V. Snieckus, *Chem. Rev.,* 1990, 90, 879), bevorzugt Halogen und C₁-C₆-Alkoxy und
- X²: eine C₁-C₂-Alkylen- oder eine C₂-Alkinylenkette bedeutet.

Geeignete metallorganische Basen zur ortho-Metallierung der literaturbekannten Benzoesäureester IIId sind z.B. Alkyllithiumverbindungen, bevorzugt n-Butyllithium oder sec-Butyllithium, Lithiumdialkylamide, bevorzugt Lithiumdiisopropylamid oder Natriumhexamethyldisilazid.

Als geeignete inerte Lösungsmittel kommen bei der direkten ortho-Metallierung z.B. Tetrahydrofuran, Diethylether, 1,2-Dimethoxyethan oder 1,4-Dioxan, Dimethylformamid, Dimethylsulfoxid, Methyl-tert-butylether oder auch Mischungen dieser Lösungsmittel in Betracht.

Die Reaktionstemperaturen können von -80 bis 100°C, vorzugsweise von -80 bis 40°C betragen.

Die Umsetzung der in situ erzeugten, ortho-metallierten Benzoesäureester IIId mit den Aldehyden V werden bei Reaktionstemperaturen von -80 bis 100°C durchgeführt.

Die erfindungsgemäßen Produkte IIIe enthalten eine Hydroxymethylen-Gruppe, die sich für weitere Derivatisierungen nach literaturbekannten Methoden eignet. So kann die Hydroxymethylen-Gruppe z.B. zur Methoxy-Gruppe methyliert werden.

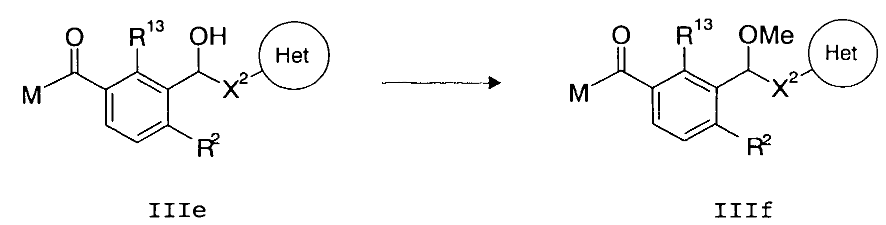

### Verfahren C:

1,3-dipolare Cycloaddition der Benzoesäureester IIIg mit gegebenenfalls substituierten Olefinen oder Acetylenen unter dehydratisierenden Bedingungen liefert die erfindungsgemäßen Benzoesäureester, wie beispielsweise IIIh.

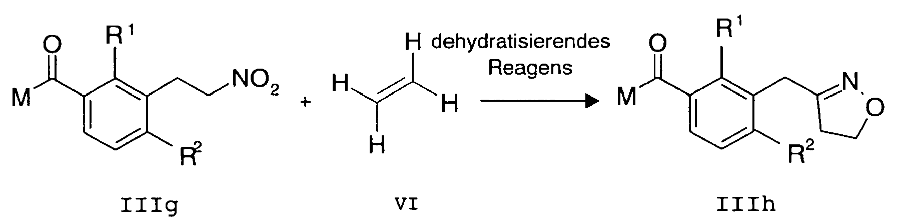

Zur Dehydratisierung nach der Methode von Mukaiyama werden bevorzugt aromatische Isocyanate (z.B. T. Mukaiyama *et al., J. Am. Chem.* Soc. 1960, 82, 5339 ), wie z.B. Phenylisocyanat oder 4-Chlorphenylisocyanat eingesetzt.

In der Variante nach Shimizu eignen sich ebenfalls aliphatische Chlorameisensäureester (z.B. T. Shimizu *et al., Bull. Chem. Soc.* Jpn. 1986, 59, 2827), bevorzugt Ethylchloroformat.

Neuere Entwicklungen zeigen, daß z.B. auch N,N-Diethylaminoschwefeltrifluorid (DAST), (Methoxycarbonylsulfamoyl)-triethylammoniumhydroxid (Burgess Reagens), Phosphorylchlorid (z.B. C. Mioskowski *et al*., *Tetrahedron Letters* 1997, *38*, 1547) oder auch eine Kombination aus Di-tert-butyl-dicarbonat (Boc₂O) und 4-Dimethylaminopyridin (DMAP) (A. Hassner *et al*., *Synthesis* 1997, 309) erfolgreich als dehydratisierende Reagenzien zur Erzeugung von Nitriloxiden eingesetzt werden können.

Die auf diese Weise in situ gebildeten Nitriloxide können bei Raumtemperatur bis zur Siedetemperatur des verwendeten Lösungsmittels mit beliebig substituierten Olefinen oder Acetylen zu den erfindungsgemäßen Benzoesäureestern IIIc umgesetzt werden, wobei hier beispielsweise X¹ eine Methylengruppe und Het einen gegebenenfalls substituiertes Isoxazol- oder Isoxazolingerüst darstellt.

Die Durchführung der Cycloaddition erfolgt in inerten Lösungsmitteln, wie beispielsweise Toluol, Chloroform oder Acetonitril.

Der Benzoesäureester IIIg kann durch Reduktion nach literaturbekannten Methoden aus IIIj erhalten werden, der durch Nitroolefinierung (z.B. a. V. V. Perekalin *et al.,* Nitroalkenes, John Wiley & Sons Ltd., New York 1994, b. A. G. M. Barrett *et al., Chem. Rev.* 1986, *86*, 751) des entsprechenden Aldehyds IIIi hergestellt werden kann.

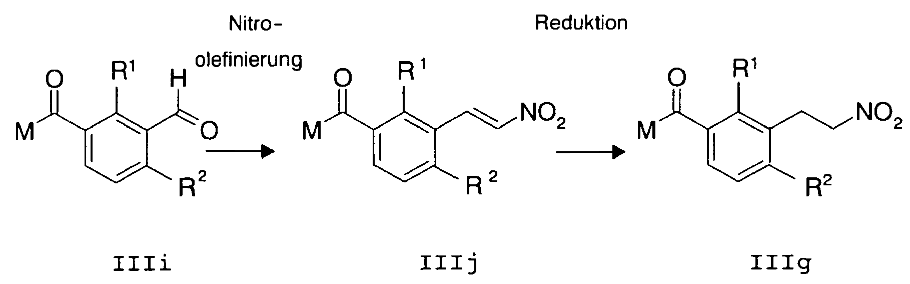

Darüber hinaus sind die erfindungsgemäßen Verbindungen der Formel I hervorzuheben, wobei die Gruppe Het für eine fünf- oder sechsgliedrige, teilweise oder vollständig gesättigte heterocyclische mit bis zu drei Heteroatomen ausgewählt aus der Gruppe Stickstoff, Sauerstoff oder Schwefel steht, wobei die genannte heterocyclische Gruppe partiell oder vollständig halogeniert und/ oder durch R⁵ substituiert sein kann;
- R⁵: Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy.

Die für die Substituenten R¹ - R¹³ oder als Reste an Phenyl-, Hetaryl- und Heterocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Alkyliminooxy-, Alkoxyamino-, Alkylsulfonyl-, Halogenalkylsulfonyl, Alkylcarbonyl-, Halogenalkylcarbonyl, Alkoxycarbonyl-, Alkoxyalkoxycarbonyl-, Alkenyl-, Cycloalkenyl-, Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl, sowie die Alkylteile von C₁-C₄-Alkylcarbonyl: Methyl, Ethyl, n-Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkoxy-C₁-C₆-alkyl und C₁-C₆-Alkylcarbonyl: C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl, sowie die Halogenalkylteile von C₁-C₆-Halogenalkylcarbonyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy, sowie die Alkoxyteile von C₁-C₄-Alkoxyamino, C₁-C₄-Alkoxy-C₂-C₆-alkoxycarbonyl und C₁-C₄-Alkoxycarbonyl: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile von C₁-C₆-Alkoxy-C₁-C₆-alkyl, C₁-C₆-Alkoxy-C₂-C₆-alkyl, C₁-C₄-Alkoxy-C₂-C₆-alkoxycarbonyl und C₁-C₆-Alkoxycarbonyl: C₁-C₄-Alkoxy wie voranstehend genannt, sowie Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z. B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Bronunethyl)-2-bromethoxy, 4-Fluorbutoxy, 4-Chlorbutoxy, 4-Brombutoxy und Nonafluorbutoxy;
- C₁-C₄-Alkylsulfonyl (C₁-C₄-Alkyl-S(=O)₂-): Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl: C₁-C₄-Alkylsulfonyl wie voranstehend genannt, sowie Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₆-Halogenalkylsulfonyl: einen C₁-C₆-Alkylsulfonylrest wie voranstehend genannt, der partiell oder vollständig durch Fluor, Chlor, Brom und/ oder Iod substituiert ist, also Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkyliminooxy: Methyliminooxy, Ethyliminooxy, 1-Propyliminooxy, 2-Propyliminooxy, 1-Butyliminooxy und 2-Butyliminooxy;
- C₃-C₆-Alkenyl: Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-l-yl, 4-Methyl-pent-2-in-4-yl und 4-Methyl-pent-2-in-5-yl;
- C₂-C₆-Alkinyl: C₃-C₆-Alkinyl, wie voranstehend genannt, sowie Ethinyl:
- C₃-C₆-Cycloalkyl: Cyclopropyl, Cyclobutyl, Cyclopentyl und Cyclohexyl;
- C₄-C₆-Cycloalkenyl: Cyclobuten-1-yl, Cyclobuten-3-yl, Cyclopenten-1-yl, Cyclopenten-3-yl, Cyclopenten-4-yl, Cyclohexen-1-yl, Cyclohexen-3-yl und Cyclohexen-4-yl;
- Heterocyclyl, sowie die Heterocyclylreste in Heterocyclyloxy: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl,
- Hetaryl, sowie die Hetarylreste in Hetaryloxy: aromatische mono- oder polycyclische Reste, welche neben Kohlenstoffringgliedern zusätzlich ein bis vier Stickstoffatome oder ein bis drei Stickstoffatome und ein Sauerstoffoder ein Schwefelatom oder ein Sauerstoff- und ein Schwefelatom enthalten können, z. B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol- 2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate.

Alle Phenyl-, Hetaryl- und Heterocyclylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen wie z.B. Fluor, Chlor oder Brom, C₁-C₆-Halogenalkyl, -OR³ oder -SO₂R³;
- R²: Wasserstoff, Nitro, Halogen wie z.B. Fluor, Chlor oder Brom, C₁-C₆-Alkyl, C₁-C₆-Halogenalkyl, -OR³ oder -SO₂R³;
- R³: Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; besonders bevorzugt Wasserstoff, C₁-C₄-Alkyl, C₁-C₄-Halogenalkyl, C₂-C₃-Alkenyl, C₂-C₃-Alkinyl oder Phenyl; wobei die genannten Alkylreste partiell oder vollständig halogeniert sein können und/oder eine bis drei der folgenden Gruppen tragen können:
Hydroxy, Mercapto, Amino, Cyano, R³, -OR³, -SR³, -N(R³)_{2,} =NOR³, -OCOR³, -SCOR³, -NR³COR³ , -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits durch ein bis drei Halogenatome und/oder einen oder zwei Reste aus folgender Gruppe: Nitro, Cyano, Methyl, Trifluormethyl, Methoxy, Trifluormethoxy oder Methoxycarbonyl substituiert sein können;
- n: 0, 1 oder 2, besonders bevorzugt 0 oder 2;
- R⁴: Wasserstoff, C₁-C₆-Alkyl oder C₁-C₆-Halogenalkyl; besonders bevorzugt Wasserstoff, Methyl, Ethyl oder Trifluormethyl;
- R⁵: C₁-C₆-Alkyl oder C₁-C₆-Halogenalkyl; besonders bevorzugt Methyl, Ethyl, Propyl, Isopropyl, Butyl oder Isobutyl;
- R⁶,: R⁷, R⁹, R¹¹ Wasserstoff, Methyl oder Ethyl;
- R⁸: besonders bevorzugt Wasserstoff, Methyl, Ethyl, Cyclopropyl, Di(methoxy)methyl, Di(ethoxy)methyl, 2-Ethylthiopropyl, Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 5,5-Dimethyl-1,3-dioxan-2-yl, 1,3-Oxathiolan-2-yl, 1,3-Oxathian-2-yl, 1,3-Dithiolan-2-yl, 5,5-Dimethyl-1,3-dithian-2-yl oder 1-Methylthiocyclopropyl;
- R¹⁰: Wasserstoff, Methyl oder Methoxycarbonyl.

Ebenso kann vorteilhaft in Betracht kommen, daß R⁸ und R¹¹ eine π-Bindung ausbilden, so daß ein Doppelbindungssystem entsteht.

Die CR⁸R⁹-Einheit kann auch vorteilhaft durch C=O ersetzt werden.

Darüber hinaus sind die erfindungsgemäßen Verbindungen der Formel Ia außerordentlich bevorzugt, in der die Substituenten R¹, R² und X¹ die oben genannte Bedeutung haben und Het für eine fünfoder sechsgliedrige, teilweise oder vollständig gesättigte heterocyclische mit bis zu drei Heteroatomen ausgewählt aus der Gruppe

Stickstoff, Sauerstoff oder Schwefel
steht.

Insbesondere bevorzugt sind die Verbindungen I der Tabellen 1 bis 36.

**Tabelle A**

| Nr. | X¹ | Het |
|---|---|---|
| 1 | CH₂ | Oxiranyl |
| 2 | CH₂ | 3-Methyl-2-oxiranyl |
| 3 | CH₂ | 2-Oxetanyl |
| 4 | CH₂ | 3-Hydroxy-3-methyl-2-oxetanyl |
| 5 | CH₂ | 3-Hydroxy-3-ethyl-2-oxetanyl |
| 6 | CH₂ | 3-Hydroxy-3-propyl-2-oxetanyl |
| 7 | CH₂ | 3-Hydroxy-3-butyl-2-oxetanyl |
| 8 | CH₂ | 3-Methoxy-3-methyl-2-oxetanyl |
| 9 | CH₂ | 3-Methoxy-3-ethyl-2-oxetanyl |
| 10 | CH₂ | 3-Methoxy-3-propyl-2-oxetanyl |
| 11 | CH₂ | 3-Methoxy-3-butyl-2-oxetanyl |
| 12 | CH₂ | 3-Trimethylsilyloxy-3-methyl-2-oxetanyl |
| 13 | CH₂ | 3-Trimethylsilyloxy-3-ethyl-2-oxetanyl |
| 14 | CH₂ | 3-Trimethylsilyloxy-3-propyl-2-oxetanyl |
| 15 | CH₂ | 3-Trimethylsilyloxy-3-butyl-2-oxetanyl |
| 16 | CH₂ | 3-Oxetanyl |
| 17 | CH₂ | 4,5-Dihydro-2-furyl |
| 18 | CH₂ | 2,3-Dihydro-2-furyl |
| 19 | CH₂ | 4,5-Dihydro-3-furyl |
| 20 | CH₂ | 2,3-Dihydro-3-furyl |
| 21 | CH₂ | 4,5-Dihydro-2-thienyl |
| 22 | CH₂ | 2,3-Dihydro-2-thienyl |
| 23 | CH₂ | 4,5-Dihydro-3-thienyl |
| 24 | CH₂ | 2,3-Dihydro-3-thienyl |
| 25 | CH₂ | 2,5-Dihydro-2-pyrrolyl |
| 26 | CH₂ | 2,5-Dihydro-3-pyrrolyl |
| 27 | CH₂ | 4,5-Dihydro-3-isoxazolyl |
| 28 | CH₂ | 4-Methyl-4,5-dihydro-3-isoxazolyl |
| 29 | CH₂ | 5-Methyl-4,5-dihydro-3-isoxazolyl |
| 30 | CH₂ | 4,5-Dimethyl-4,5-dihydro-3-isoxazolyl |
| 31 | CH₂ | 4,5-Dihydro-4-isoxazolyl |
| 32 | CH₂ | 3-Methyl-4,5-dihydro-4-isoxazolyl |
| 33 | CH₂ | 5-Methyl-4,5-dihydro-4-isoxazolyl |
| 34 | CH₂ | 3,5-Dimethyl-4,5-dihydro-4-isoxazolyl |
| 35 | CH₂ | 4,5-Dihydro-5-isoxazolyl |
| 36 | CH₂ | 3-Methyl-4,5-dihydro-5-isoxazolyl |
| 37 | CH₂ | 4-Methyl-4,5-dihydro-5-isoxazolyl |
| 38 | CH₂ | 3,4-Dimethyl-4,5-dihydro-5-isoxazolyl |
| 39 | CH₂ | 2-(2*H*-1,3-oxazinyl) |
| 40 | CH₂ | 2-(6*H*-1,3-oxazinyl) |
| 41 | CH₂ | 4-(6*H*-1,3-oxazinyl) |
| 42 | CH₂ | 6-(6*H*-1,3-oxazinyl) |
| 43 | CH₂CH₂ | Oxiranyl |
| 44 | CH₂CH₂ | 3-Methyl-2-oxiranyl |
| 45 | CH₂CH₂ | 2-Oxetanyl |
| 46 | CH₂CH₂ | 3-Hydroxy-3-methyl-2-oxetanyl |
| 47 | CH₂CH₂ | 3-Hydroxy-3-ethyl-2-oxetanyl |
| 48 | CH₂CH₂ | 3-Hydroxy-3-propyl-2-oxetanyl |
| 49 | CH₂CH₂ | 3-Hydroxy-3-butyl-2-oxetanyl |
| 50 | CH₂CH₂ | 3-Methoxy-3-methyl-2-oxetanyl |
| 51 | CH₂CH₂ | 3-Methoxy-3-ethyl-2-oxetanyl |
| 52 | CH₂CH₂ | 3-Methoxy-3-propyl-2-oxetanyl |
| 53 | CH₂CH₂ | 3-Methoxy-3-butyl-2-oxetanyl |
| 54 | CH₂CH₂ | 3-Trimethylsilyloxy-3-methyl-2-oxetanyl |
| 55 | CH₂CH₂ | 3-Trimethylsilyloxy-3-ethyl-2-oxetanyl |
| 56 | CH₂CH₂ | 3-Trimethylsilyloxy-3-propyl-2-oxetanyl |
| 47 | CH₂CH₂ | 3-Trimethylsilyloxy-3-butyl-2-oxetanyl |
| 58 | CH₂CH₂ | 3-Oxetanyl |
| 59 | CH₂CH₂ | 4,5-Dihydro-2-furyl |
| 60 | CH₂CH₂ | 2,3-Dihydro-2-furyl |
| 61 | CH₂CH₂ | 4,5-Dihydro-3-furyl |
| 62 | CH₂CH₂ | 2,3-Dihydro-3-furyl |
| 63 | CH₂CH₂ | 4,5-Dihydro-2-thienyl |
| 64 | CH₂CH₂ | 2,3-Dihydro-2-thienyl |
| 65 | CH₂CH₂ | 4,5-Dihydro-3-thienyl |
| 66 | CH₂CH₂ | 2,3-Dihydro-3-thienyl |
| 66 | CH₂CH₂ | 2,5-Dihydro-2-pyrrolyl |
| 68 | CH₂CH₂ | 2,5-Dihydro-3-pyrrolyl |
| 69 | CH₂CH₂ | 4,5-Dihydro-3-isoxazolyl |
| 70 | CH₂CH₂ | 4-Methyl-4,5-dihydro-3-isoxazolyl |
| 71 | CH₂CH₂ | 5-Methyl-4,5-dihydro-3-isoxazolyl |
| 72 | CH₂CH₂ | 4,5-Dimethyl-4,5-dihydro-3-isoxazolyl |
| 73 | CH₂CH₂ | 4,5-Dihydro-4-isoxazolyl |
| 74 | CH₂CH₂ | 3-Methyl-4,5-dihydro-4-isoxazolyl |
| 75 | CH₂CH₂ | 5-Methyl-4,5-dihydro-4-isoxazolyl |
| 76 | CH₂CH₂ | 3,5-Dimethyl-4,5-dihydro-4-isoxazolyl |
| 77 | CH₂CH₂ | 4,5-Dihydro-5-isoxazolyl |
| 78 | CH₂CH₂ | 3-Methyl-4,5-dihydro-5-isoxazolyl |
| 79 | CH₂CH₂ | 4-Methyl-4,5-dihydro-5-isoxazolyl |
| 80 | CH₂CH₂ | 3,4-Dimethyl-4,5-dihydro-5-isoxazolyl |
| 81 | CH₂CH₂ | 2-(2*H*-1,3-oxazinyl) |
| 82 | CH₂CH₂ | 2-(6*H*-1,3-oxazinyl) |
| 83 | CH₂CH₂ | 4-(6*H*-1,3-oxazinyl) |
| 84 | CH₂CH₂ | 6-(6*H*-1,3-oxazinyl) |
| 85 | -C≡C- | Oxiranyl |
| 86 | -C≡C- | 3-Methyl-2-oxiranyl |
| 87 | -C≡C- | 2-Oxetanyl |
| 88 | -C≡C- | 3-Hydroxy-3-methyl-2-oxetanyl |
| 89 | -C≡C- | 3-Hydroxy-3-ethyl-2-oxetanyl |
| 90 | -C≡C- | 3-Hydroxy-3-propyl-2-oxetanyl |
| 91 | -C≡C- | 3-Hydroxy-3-butyl-2-oxetanyl |
| 92 | -C≡C- | 3-Methoxy-3-methyl-2-oxetanyl |
| 93 | -C≡C- | 3-Methoxy-3-ethyl-2-oxetanyl |
| 94 | -C≡C- | 3-Methoxy-3-propyl-2-oxetanyl |
| 95 | -C≡C- | 3-Methoxy-3-butyl-2-oxetanyl |
| 96 | -C≡C- | 3-Trimethylsilyloxy-3-methyl-2-oxetanyl |
| 97 | -C≡C- | 3-Trimethylsilyloxy-3-ethyl-2-oxetanyl |
| 98 | -C≡C- | 3-Trimethylsilyloxy-3-propyl-2-oxetanyl |
| 99 | -C≡C- | 3-Trimethylsilyloxy-3-butyl-2-oxetanyl |
| 100 | -C≡C- | 3-Oxetanyl |
| 101 | -C≡C- | 4,5-Dihydro-2-furyl |
| 102 | -C≡C- | 2,3-Dihydro-2-furyl |
| 103 | -C≡C- | 4,5-Dihydro-3-furyl |
| 104 | -C≡C- | 2,3-Dihydro-3-furyl |
| 105 | -C≡C- | 4,5-Dihydro-2-thienyl |
| 106 | -C≡C- | 2,3-Dihydro-2-thienyl |
| 107 | -C≡C- | 4,5-Dihydro-3-thienyl |
| 108 | -C≡C- | 2,3-Dihydro-3-thienyl |
| 109 | -C≡C- | 2,5-Dihydro-2-pyrrolyl |
| 110 | -C≡C- | 2,5-Dihydro-3-pyrrolyl |
| 111 | -C≡C- | 4,5-Dihydro-3-isoxazolyl |
| 112 | -C≡C- | 4-Methyl-4,5-dihydro-3-isoxazolyl |
| 113 | -C≡C- | 5-Methyl-4,5-dihydro-3-isoxazolyl |
| 114 | -C≡C- | 4,5-Dimethyl-4,5-dihydro-3-isoxazolyl |
| 115 | -C≡C- | 4,5-Dihydro-4-isoxazolyl |
| 116 | -C≡C- | 3-Methyl-4,5-dihydro-4-isoxazolyl |
| 117 | -C≡C- | 5-Methyl-4,5-dihydro-4-isoxazolyl |
| 118 | -C≡C- | 3,5-Dimethyl-4,5-dihydro-4-isoxazolyl |
| 119 | -C≡C- | 4,5-Dihydro-5-isoxazolyl |
| 120 | -C≡C- | 3-Methyl-4,5-dihydro-5-isoxazolyl |
| 121 | -C≡C- | 4-Methyl-4,5-dihydro-5-isoxazolyl |
| 122 | -C≡C- | 3,4-Dimethyl-4,5-dihydro-5-isoxazolyl |
| 123 | -C≡C- | 2-(2*H*-1,3-oxazinyl) |
| 124 | -C≡C- | 2-(6*H*-1,3-oxazinyl) |
| 125 | -C≡C- | 4-(6*H*-1,3-oxazinyl) |
| 126 | -C≡C- | 6-(6*H*-1,3-oxazinyl) |

Die folgenden Tabellen 1 - 36 basieren auf den 2-Benzoyl-cyclohexan-1,3-dionen der Formel I

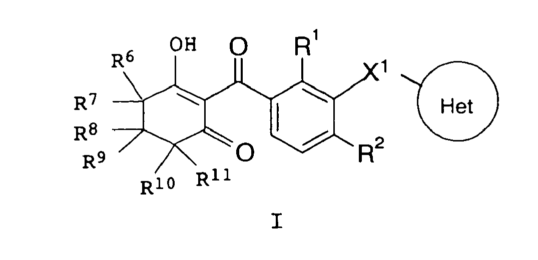

### Tabelle 1: Verbindungen 1.1-1.126

Verbindungen der allgemeinen Formel I, in der R¹ Chlor, R² Methylsulfonyl, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 2: Verbindungen 2.1-2.126

Verbindungen der allgemeinen Formel I, in der R¹ und R² Chlor, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 3: Verbindungen 3.1-3.126

Verbindungen der allgemeinen Formel I, in der R¹ Chlor, R² Trifluormethyl, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 4: Verbindungen 4.1-4.126

Verbindungen der allgemeinen Formel I, in der R¹ Methyl, R² Chlor, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 5: Verbindungen 5.1-5.126

Verbindungen der allgemeinen Formel I, in der R¹ Methyl, R² Methylsulfonyl, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 6: Verbindungen 6.1-6.126

Verbindungen der allgemeinen Formel I, in der R¹ Methyl, R² Trifluormethyl, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 7: Verbindungen 7.1-7.126

Verbindungen der allgemeinen Formel I, in der R¹ Chlor, R² Methylsulfonyl, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 8: Verbindungen 8.1-8.126

Verbindungen der allgemeinen Formel I, in der R¹ und R² Chlor, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 9: Verbindungen 9.1-9.126

Verbindungen der allgemeinen Formel I, in der R¹ Chlor, R² Trifluormethyl, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 10: Verbindungen 10.1-10.126

Verbindungen der allgemeinen Formel I, in der R¹ Methyl, R² Chlor, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 11: Verbindungen 11.1-11.126

Verbindungen der allgemeinen Formel I, in der R¹ Methyl, R² Methylsulfonyl, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 12: Verbindungen 12.1-12.126

Verbindungen der allgemeinen Formel I, in der R¹ Methyl, R² Trifluormethyl, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 13: Verbindungen 13.1-13.126

Verbindungen der allgemeinen Formel I, in der R¹ Chlor, R² Methylsulfonyl, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 14: Verbindungen 14.1-14.126

Verbindungen der allgemeinen Formel I, in der R¹ und R² Chlor, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 15: Verbindungen 15.1-15.126

Verbindungen der allgemeinen Formel I, in der R¹ Chlor, R² Trifluormethyl, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 16: Verbindungen 16.1-16.126

Verbindungen der allgemeinen Formel I, in der R¹ Methyl, R² Chlor, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 17: Verbindungen 17.1-17.126

Verbindungen der allgemeinen Formel I, in der R¹ Methyl, R² Methylsulfonyl, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 18: Verbindungen 18.1-18.126

Verbindungen der allgemeinen Formel I, in der R¹ Methyl, R² Trifluormethyl, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 19: Verbindungen 19.1-19.126

Verbindungen der allgemeinen Formel I, in der R¹ Chlor, R² Methylsulfonyl, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 20: Verbindungen 20.1-20.126

Verbindungen der allgemeinen Formel I, in der R¹ und R² Chlor, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 21: Verbindungen 21.1-21.126

Verbindungen der allgemeinen Formel I, in der R¹ Chlor, R² Trifluormethyl, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 22: Verbindungen 22.1-22.126

Verbindungen der allgemeinen Formel I, in der R¹ Methyl, R² Chlor, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 23: Verbindungen 23.1-23.126

Verbindungen der allgemeinen Formel I, in der R¹ Methyl, R² Methylsulfonyl, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 24: Verbindungen 24.1-24.126

Verbindungen der allgemeinen Formel I, in der R¹ Methyl, R² Trifluormethyl, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 25: Verbindungen 25.1-25.126

Verbindungen der allgemeinen Formel I, in der R¹ Methylsulfonyl, R⁶, R⁷, R⁸, R¹⁰ und R¹¹ Wasserstoff, R⁹ Methyl bedeutet, die Substituenten X¹ und Het für jede einzelne Verbindung jeweils einer Zeile der Tabelle A entsprechen.

### Tabelle 26: Verbindungen 26.1-26.126

Verbindungen der allgemeinen Formel I, in der R¹ und R² Chlor, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 27: Verbindungen 27.1-27.126

Verbindungen der allgemeinen Formel I, in der R¹ Chlor, R² Trifluormethyl, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 28: Verbindungen 28.1-28.126

Verbindungen der allgemeinen Formel I, in der R¹ Methyl, R² Chlor, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 29: Verbindungen 29.1-29.126

Verbindungen der allgemeinen Formel I, in der R¹ Methyl, R² Methylsulfonyl, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 30: Verbindungen 30.1-30.126

Verbindungen der allgemeinen Formel I, in der R¹ Methyl, R² Trifluormethyl, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 31: Verbindungen 31.1-31.126

Verbindungen der allgemeinen Formel I, in der R¹ Chlor, R² Methylsulfonyl, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 32: Verbindungen 32.1-32.126

Verbindungen der allgemeinen Formel I, in der R¹ und R² Chlor, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 33: Verbindungen 33.1-33.126

Verbindungen der allgemeinen Formel I, in der R¹ Chlor, R² Trifluormethyl, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 34: Verbindungen 34.1-34.126

Verbindungen der allgemeinen Formel I, in der R¹ Methyl, R² Chlor, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 35: Verbindungen 35.1-35.126

Verbindungen der allgemeinen Formel I, in der R¹ Methyl, R² Methylsulfonyl, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 36: Verbindungen 36.1-36.126

Verbindungen der allgemeinen Formel I, in der R¹ Methyl, R² Trifluormethyl, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

### Als inerte Zusatzstoffe kommen im wesentlichen in Betracht:

Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die substituierte 2-Benzoylcyclohexan-1,3-dione als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta-und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentration der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, minestens eines Wirkstoffs. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
- I: 20 Gewichtsteile der Verbindung Nr. 20.27 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
- II: 20 Gewichtsteile der Verbindung Nr. 20.27 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- III: 20 Gewichtsteile der Verbindung Nr.20.27 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- IV: 20 Gewichtsteile der Verbindung Nr. 20.27 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
- V: 3 Gewichtsteile der Verbindung Nr. 20.27 werden mit 97 Gewichtsteilen feinteiligem Kaolin vermischt. Man erhält auf diese Weise ein Stäubemittel, das 3 Gew.-% des Wirkstoffs enthält.
- VI: 20 Gewichtsteile der Verbindung Nr. 20.27 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII: 1 Gewichtsteil der Verbindung Nr. 20.27 wird in einer Mischung gelöst, die aus 70 Gewichtsteilen Cyclohexanon, 20 Gewichtsteilen ethoxyliertem Isooctylphenol und 10 Gewichtsteilen ethoxyliertem Ricinusöl besteht. Man erhält ein stabiles Emulsionskonzentrat.
- VIII: 1 Gewichtsteil der Verbindung Nr. 20.27 wird in einer Mischung gelöst, die aus 80 Gewichtsteilen Cyclohexanon und 20 Gewichtsteilen Wettol ® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die substituierte 2-Benzoylcyclohexan-1,3-dione mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazone, 2-Aroyl-1,3-cyclohexandione, Hetaryl-Aryl-Ketone, Benzylisooxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Nachfolgend werden die Synthesen einiger Edukte und Produkte beschrieben.

### 2-Chlor-3-((4,5-dihydro-3-isoxazolylmethyl)-phenyl)-4-sulfonylmethyl-(5,5-dimethyl-cyclohexan-1,3-dion)-methanon

### Stufe a: 2-Chlor-3-(2-nitroethenyl)-4-sulfonylmethyl-benzoesäuremethylester

50 g (0,18 mol) 2-Chlor-3-formyl-4-sulfonylmethyl-benzoesäuremethylester, 220 g (3,6 mol) Nitromethan, 216 g (3,6 mol) Eisessig und 7 g (91 mmol) Ammoniumacetat werden unter Ausschluß von Luftfeuchtigkeit bei 85°C gerührt. Das Reaktionsgemisch wird auf 2 1 dest. Wasser gegossen und mit Diethylether extrahiert. Die organische Phase wird mit gesättigter, wäßriger Natriumhydrogencarbonat-Lösung gewaschen, mit Natriumsulfat getrocknet, filtriert und i. Vak. vom Lösungsmittel befreit. Das Rohprodukt wird aus *n*-Hexan/Essigsäureethylester umkristallisiert. Ausbeute: 13,1 g, Fp. 147 - 150°C

### Stufe b: 2-Chlor-3-(2-nitroethyl)-4-sulfonylmethyl-benzoesäuremethylester

19,4 g (61 mmol) 2-Chlor-3-(2-nitroethenyl)-4-sulfonylmethyl-benzoesäuremethylester und 121 g Kieselgel 60 werden in 180 ml 2-Propanol und 970 ml Chloroform suspendiert und bei Raumtemp. mit 3.5 g (94 mmol) Natriumborhydrid behandelt. Das Reaktionsgemisch wird filtriert, das Filtrat mit Dichlormethan/ Methanol 1:1 (v/v) gewaschen und die organische Phase i. Vak. vom Lösungsmittel befreit. Ausbeute: 19,5 g; ¹H-NMR, δ [ppm], CDCl₃: 3.2 (s), 3,9 (m), 4,0 (s), 4,7 (m), 7,8 (d), 8,1 (d)

### Stufe c: 2-Chlor-3-((4,5-dihydro-3-isoxazolylmethyl)-4-sulfonylmethyl-benzoesäuremethylester

Eine Lösung aus 2,8 g (23 mmol) Phenylisocyanat in 50 ml Toluol wird bei Raumtemp. mit Ethylen begast. Dazu wird eine Lösung aus 3,7 g (11 mmol) 2-Chlor-3-(2-nitroethyl)-4-sulfonylmethyl-benzoesäuremethylester und 0,5 ml Triethylamin in 100 ml Toluol gegeben. Das Reaktionsgemisch wird in 300 ml Diethylether aufgenommen, mit dest. Wasser extrahiert, mit Natriumsulfat getrocknet , filtriert und i. Vak vom Lösungsmittel befreit. Das Rohprodukt wird an Kieselgel 60 mit Cyclohexan/Essigsäureethylester gereinigt. Ausbeute: 1.0 g; ¹H-NMR, δ [ppm], CDCl₃: 3,1 (t), 3,2 (s), 4,0 (s), 4,4 (t), 4,5 (s), 7,7 (d), 8,1 (d)

### Stufe d: 2-Chlor-3-((4,5-dihydro-3-isoxazolylmethyl)-4-sulfonylmethyl-benzoesäure

Eine Lösung aus 2,3 g (7 mmol) 2-Chlor-3-((4,5-dihydro-3-isoxazolylmethyl)-4-sulfonylmethyl-benzoesäuremethylester in 40 ml Tetrahydrofuran wird bei 5°C mit 0,3 g (13 mmol) Lithiumhydroxid behandelt. Das Reaktionsgemisch wird in 300 ml Diethylether aufgenommen und mit 10 %iger, wäßriger Salzsäure-Lösung auf pH 1 eingestellt. Die wäßrige Phase wird mit Diethylether extrahiert. Die vereinigten organischen Phasen werden über Natriumsulfat getrocknet, filtriert und i. Vak. vom Lösungsmittel befreit. Ausbeute 1,7 g; ¹H-NMR, δ [ppm], DMSO-d₆: 3,0 (t), 3,3 (s), 4,2 (t), 4,3 (s), 7,8 (d), 8,1 (d)

### Stufe e: 2-Chlor-3-((4,5-dihydro-3-isoxazolylmethyl)-phenyl)-4-sulfonylmethyl-(5,5-dimethyl-cyclohexan-1,3-dion)-methanon (Tabelle 7, Beispiel 27)

Eine Lösung aus 1,0 g (3 mmol) 2-Chlor-3-((4,5-dihydro-3-isoxazolylmethyl)-4-sulfonylmethyl-benzoesäure und 440 mg (3 mmol) Dimedon in 50 ml Acetonitril wird bei 40°C mit 0,8 g (3.8 mmol) *N,N*-Dicyclohexylcarbodiimid behandelt. Nach abgeschlossener Reaktion (DC-Kontrolle) gibt man 310 mg (3 mmol) Trimethylsilylcyanid und 950 mg (9 mmol) Triethylamin zu. Das Reaktionsgemisch wird filtriert und i. Vak. vom Lösungsmittel befreit. Das Rohprodukt wird an Kieselgel 60 mit Toluol/ Tetrahydrofuran oder Dichlormethan/Toluol gereinigt. Ausbeute 600 mg; Fp. 168 - 173°C

Die im folgenden beschriebenen Verbindung wurden analog zu den oben angegebenen Arbeitsvorschriften dargestellt.

2-Chlor-3-((4,5-dihydro-3-isoxazolylmethyl)-phenyl)-4-sulfonylmethyl-(cyclohexan-1,3-dion)-methanon (Tabelle 1, Beispiel 27)
Fp. 70 - 74°C

2-Chlor-3-((4,5-dihydro-3-isoxazolylmethyl)-phenyl)-4-sulfonylmethyl-(3,3,6,6-tetramethyl-cyclohexan-1,3,5-trion)-methanon (Tabelle 19, Beispiel 27)
Fp. 168 - 173°C

2,4-Dichlor-3-((4,5-dihydro-3-isoxazolylmethyl)-phenyl)-(5,5-dimethyl-cyclohexan-1,3-dion)-methanon (Tabelle 8, Beispiel 27)
Fp. 56 - 64°C

2,4-Dichlor-3-((4,5-dihydro-3-isoxazolylmethyl)-phenyl)-(3,3,6,6-tetramethyl-cyclohexan-1,3,5-trion)-methanon (Tabelle 20, Beispiel 27)
Fp. 196 - 202°C

2,4-Dichlor-3-((3-(5H-furanon)methyl)phenyl)-2,2,4,4,-tetramethyl-1,3,5-cyclohexantrion)methanon
- Stufe a:: 2,4-Dichlor-3-(3-(5H-furanon)methyl)benzoesäure Die Lösung von 13 g (0,038 mol)
2,4-Dichlor-3-(3-furyl)hydroxymethylbenzoesäure-tert-butylester und 1,8 g p-Toluolsulfonsäure in 370 ml Toluol werden 6h refluxiert. Anschließend wird auf 100 ml 10 % Natronlauge gegeben und mit Essigester extrahiert. Die wässrige Phase wird mit Salzsäure angesäuert und mit Essigester mehrmals extrahiert. Die vereinigten organischen Phasen werden mit Wasser gewaschen, getrocknet und eingeengt. Es verbleiben 4,8 g (45 %) der Titelverbindung Fp.196°C
- Stufe b:: 2,4-Dichlor-3-((3-(5H-furanon)methyl)phenyl)-(2,2,4,4-tetramethyl-1,3,5-cyclohexantrion)methanon 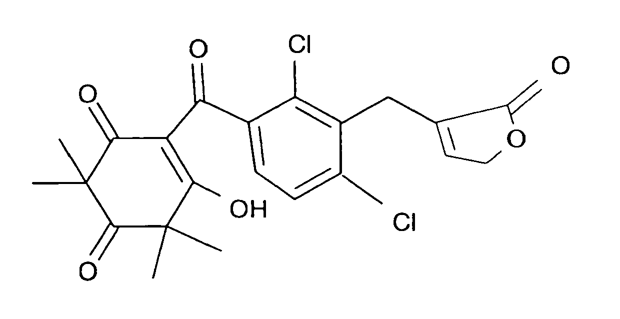 1,6 g (0,0056 mol) der so erhaltenen Säure, 1,0 g (0,0056 mol) 2,2,4,4-tetramethyl-1,3,5-cyclohexantrion und 1,15 g (0,0056 mol) Dicyclohexylcarbodiimid werden bei Raumtemperatur in 15 ml Acentonitril gerührt. Anschließend werden 1,13 g (0,012 mol) Triethylamin und 0,1 ml Trimethylsilylcyanid zugegeben und weitere 12h bei Raumtemperatur belassen.
Der Reaktionsansatz wird mit 50 ml Essigester verdünnt, mit 50 ml Natriumcarbonat-Lösung versetzt und der entstandene Niederschlag verworfen. Die organische Phase wird abgetrennt, getrocknet und eingeengt. Der erhaltene Rückstand wird an einer Kieselgelsäule chromatographiert (Cyclohexan/Essigester 1:10). Man erhält 120 mg (5 %) der Titelverbindung.
(charakteristische 13-C -NMR-Daten: 209.1, 197.6, 195.9, 195.1, 173.4, 145.6, 137.7, 136.2, 134.7, 131.3, 130.6, 128.6, 126.2, 110.2, 70.5, 27.7,
In analoger Weise wurde hergestellt: 2,4-Dichlor-3-((3-(5H-furanon)methyl)phenyl)-(1,3-cyclohexandion-2yl-)methanon Schmelzpunkt: 119-121

**Tabelle 37**

| 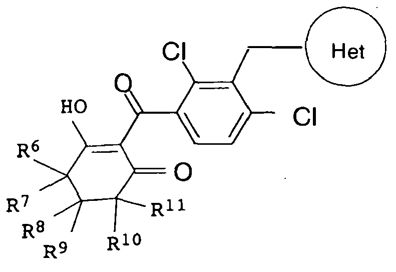 | | | | | | | | | |
|---|---|---|---|---|---|---|---|---|---|
| Nr. | R⁶ | R⁷ | R⁸ | R⁹ | R¹⁰ | R¹¹ | Het | Fp. [°C] | ¹H-NMR [ppm] |
| 37.1 | H | H | CH₃ | CH₃ | H | H | 4,5-Dihydro-3-isoxazolyl | 64 | |
| 37.2 | CH₃ | CH₃ | C=O | | CH₃ | CH₃ | 4,5-Dihydro-3-isoxazolyl | 202 | |

**Tabelle 38**

| | | | | | | | | | |
|---|---|---|---|---|---|---|---|---|---|
| 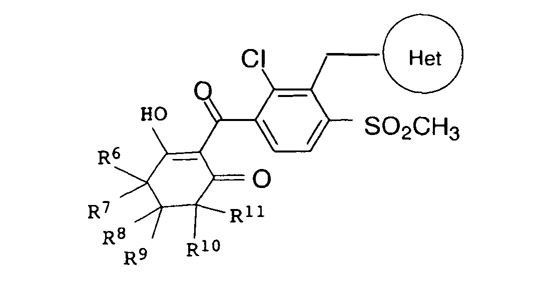 | | | | | | | | | |

| Nr. | R⁶ | R⁷ | R⁸ | R⁹ | R¹⁰ | R¹¹ | Het | Fp. [°C] | ¹H-NMR [ppm] |
|---|---|---|---|---|---|---|---|---|---|
| 38.1 | H | H | H | H | H | H | 4,5-Dihydro-3-isoxazolyl | 74 | |
| 38.2 | H | H | CH₃ | CH₃ | H | H | 4,5-Dihydro-3-isoxazolyl | 96 | |
| 38.3 | H | H | CH₃ | CH₃ | H | H | 4,5-Dihydro-5-methyl-3-isoxazolyl | 119 | |
| 38.4 | CH₃ | CH₃ | C=O | | CH₃ | CH₃ | 4,5-Dihydro-3-isoxazolyl | 173 | |
| 38.5 | CH₃ | CH₃ | C=O | | CH₃ | CH₃ | 4,5-Dihydro-5-chlor-methyl-3-isoxazolyl | 84 | |

### Anwendungsbeispiele

Die herbizide Wirkung der substituierte 2-Benzoylcyclohexan-1,3-dione der Formel I ließ sich durch Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0.5 bzw. 0.25 kg/ha a. S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Chenopodium album | Weißer Gänsefuß | lambsquarters (goosefoot) |
| Setaria viridis | Grüne Borstenhirse | green foxtail |
| Sinapis alba | Weißer Senf | white mustard |
| Solanum nigrum | Schwarzer Nachtschatten | black nightshade |

Herbizide Aktivität bei Nachauflaufanwendung im Gewächshaus

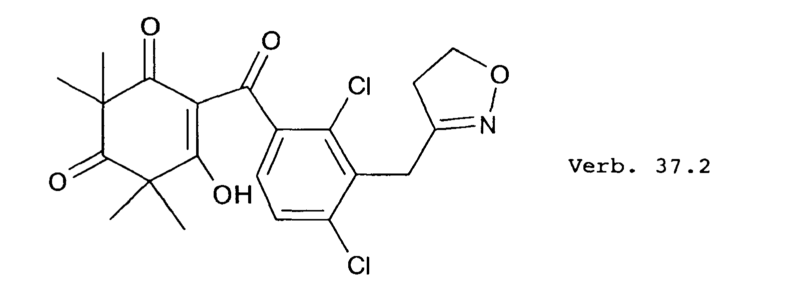

Oben genannte Unkräuter werden von der Verbindung 37.2 im Nachauflauf bei Aufwandmengen von 0,5 bzw. 0,25 kg/ha a.S. sehr gut bekämpft.

## Patentansprüche

1. 2-Benzyol-cyclohexan-1,3-dione der Formel I 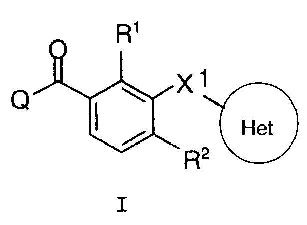 in der die Substituenten folgende Bedeutung haben:
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR³ oder -S(O)ₙR³ bedeutet;
R² für Wasserstoff oder einen wie voranstehend unter R¹ genannten Rest steht.
R³ Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/ oder eine bis drei der folgenden Gruppen tragen können:
Hydroxy, Mercapto, Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits durch ein bis drei Halogenatome und/ oder einen oder zwei Reste aus folgender Gruppe: Nitro, Cyano, Methyl, Trifluormethyl, Methoxy, Trifluormethoxy oder Methoxycarbonyl substituiert sein können;
n 0, 1 oder 2;
Q ein in 2-Stellung verknüpfter Cyclohexan-1,3-dion-Ring der Formel II 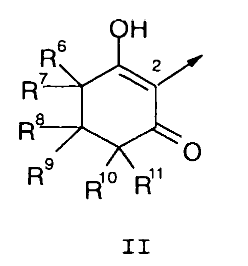
wobei R⁶, R⁷, R⁹ und R¹¹ für Wasserstoff oder C₁-C₄-Alkyl stehen;
R⁸ für Wasserstoff, C₁-C₄-Alkyl oder C₃-C₄-Cycloalkyl steht, wobei die beiden letztgenannten Gruppen einen bis drei der folgenden Substituenten tragen können:
Halogen, C₁-C₄-Alkylthio oder C₁-C₄-Alkoxy;
oder
für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch einen bis drei C₁-C₄-Alkylreste substituiert sein können;
R¹⁰ für Wasserstoff, C₁-C₄-Alkyl oder C₁-C₆-Alkoxycarbonyl steht;
oder
R⁸ und R¹¹ gemeinsam eine p-Bindung oder einen drei- bis sechsgliedrigen carbocyclischen Ring bilden;
oder
die CR⁸R⁹-Einheit durch C=O ersetzt sein kann;
X¹ eine C₁-C₂-Alkylen- oder eine C₂-Alkinylenkette;,
R⁴ Wasserstoff, C₁-C₆-Alkyl, C₂-C₆-Alkenyl, C₂-C₆-Alkinyl, Phenyl, Phenyl-C₁-C₆-alkyl, wobei die genannten Alkyl-, Alkenyl oder Alkinylreste partiell oder vollständig halogeniert sein können und/ oder durch einen oder mehrere der folgenden Reste substituiert sein können:
Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
Het eine drei- bis sechsgliedrige, teilweise oder vollständig gesättigte, heterocyclische Gruppe mit bis zu drei Heteroatomen ausgewählt aus der Gruppe:
Stickstoff, Sauerstoff oder Schwefel,
wobei die genannte heterocyclische Gruppe partiell oder vollständig halogeniert sein kann und/oder durch R⁵ substituiert sein kann;
R⁵ Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
sowie deren landwirtschaftlich brauchbaren Salze.

2. 2-Benzoyl-cyclohexan-1,3-dione der Formel I nach einem der Ansprüche 1 bis 2, in der Het eine fünf- oder sechsgliedrige, teilweise oder vollständig gesättigte heterocyclische mit bis zu drei Heteroatomen ausgewählt aus der Gruppe
Stickstoff, Sauerstoff oder Schwefel
steht.

3. Verfahren zur Herstellung von 2-Benzoyl-cyclohexan-1,3-dienen der Formel I gemäß den Ansprüchen 1 bis 2, **dadurch gekennzeichnet, daß** man ein gegebenenfalls substituiertes Cyclohexan-1,3-dion Q mit einer aktivierten Carbonsäure IIIa oder mit einer Carbonsäure IIIb, 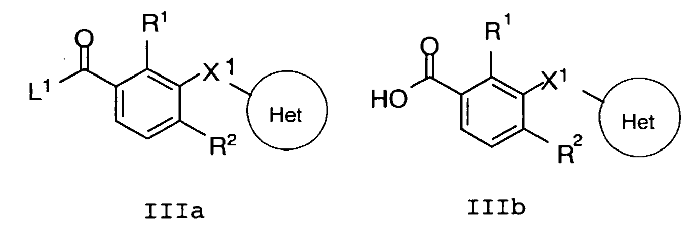 wobei die Substituenten R¹, R², X¹ und Het die in Anspruch 1 genannte Bedeutung haben und L¹ für eine nucleophil austauschbare Abgangsgruppe steht, acyliert und das Acylierungsprodukt gegebenenfalls in Gegenwart eines Katalysators zu den Verbindungen I umlagert.

4. Aktivierte Carbonsäuren der Formel IIIa und Carbonsäuren der Formel IIIb gemäß Anspruch 3, wobei die Substituenten R¹, R², X¹ und Het die in Anspruch 1 genannte Bedeutung haben und L¹ für eine nucleophil austauschbare Abgangsgruppe steht.

5. Mittel, enthaltend eine herbizid wirksame Menge mindestens eines 2-Benzoyl-cyclohexan-1,3-dions der Formel I oder eines landwirtschaftlich brauchbaren Salzes von I gemäß den Ansprüchen 1 bis 2 und für die Formulierung von Pflanzenschutzmitteln übliche Hilfsmittel.

6. Verfahren zur Herstellung von herbizid wirksamen Mitteln gemäß Anspruch 5, **dadurch gekennzeichnet, daß** man eine herbizid wirksame Menge mindestens eines 2-Benzoyl-cyclohexan-1,3-dions der Formel I oder eines landwirtschaftlich brauchbaren Salzes von I gemäß den Ansprüchen 1 bis 2 und für die Formulierung von Pflanzenschutzmitteln übliche Hilfsmittel mischt.

7. Verfahren zur Bekämpfung von unerwünschtem Pflanzenwuchs, **dadurch gekennzeichnet, daß** man eine herbizid wirksame Menge mindestens eines 2-Benzoyl-cyclohexan-1,3-dions der Formel I oder eines landwirtschaftlich brauchbaren Salzes von I gemäß den Ansprüchen 1 bis 2 auf Pflanzen, deren Lebensraum und/ oder auf Samen einwirken läßt.

8. Verwendung der 2-Benzoyl-cyclohexan-1,3-dione der Formel I und deren landwirtschaftlich brauchbaren Salze gemäß den Ansprüchen 1 bis 2 als Herbizide.

## Claims

1. A 2-benzoylcyclohexan-1,3-dione of the formula I 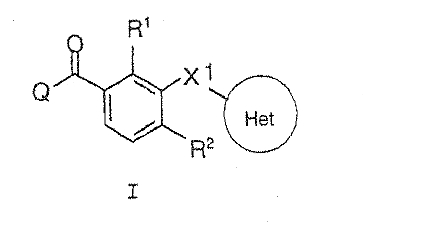 where:
R¹ is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR³ or -S(O)ₙR³;
R² is hydrogen or one of the radicals mentioned above under R¹.
R³ is hydrogen, C₁-C₆-alkyl, C₁-C₆-haloalkyl, C₂-C₆-alkenyl, C₂-C₆-alkynyl, phenyl or phenyl-C₁-C₆-alkyl; where the alkyl radicals mentioned may be partially or fully halogenated and/or carry one to three of the following groups:
hydroxyl, mercapto, amino, cyano, R³, -OR³, - SR³, -N (R³)₂, =NOR³, - OCOR³, -SCOR³, -NR³COR³, -CO₂R³, -COSR³, -CON(R³)₂, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkyl-sulfonyl, heterocyclyl, heterocyclyloxy, phenyl, benzyl, hetaryl, phenoxy, benzyloxy and hetaryloxy, where the eight last-mentioned radicals may in turn be substituted by one to three halogen atoms and/or one or two radicals from the following group: nitro, cyano, methyl, trifluoromethyl, methoxy, trifluoromethoxy or methoxycarbonyl;
n is 0, 1 or 2;
Q is a cyclohexan-1,3-dione ring of the formula II which is attached in position 2, 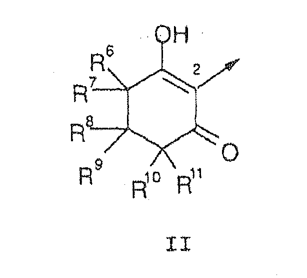
where R⁶, R⁷, R⁹ and R¹¹ are hydrogen or C₁-C₄-alkyl;
R⁸ is hydrogen, C₁-C₄-alkyl or C₃-C₄-cycloalkyl,
where the two last-mentioned groups may carry one to three of the following substitutents:
halogen, C₁-C₄-alkylthio or C₁-C₄-alkoxy;
or
is tetrahydropyran-2-yl, tetrahydropyran-3-yl, tetrahydropyran-4-yl, tetrahydrothiopyran-2-yl, tetrahydrothiopyran-3-yl, tetrahydrothiopyran-4-yl, 1,3-dioxolan-2-yl, 1,3-di-oxan-2-yl, 1,3-oxathiolan-2-yl, 1,3-oxathian-2-yl, 1,3-dithiolan-2-yl or 1,3-dithian-2-yl, where the 6 last-mentioned radicals may be substituted by one to three C₁-C₄-alkyl radicals;
R¹⁰ is hydrogen, C₁-C₄-alkyl or C₁-C₆-alkoxy-carbonyl;
or
R⁸ and R¹¹ together form a p-bond or a three- to six-membered carbocyclic ring;
or
the CR⁸R⁹ unit may be replaced by C=O;
X¹ is a C₁-C₂-alkylene or a C₁-C₂-alkynylene chain;
R⁴ is hydrogen, C₁-C₆-alkyl, C₂-C₆-alkenyl, C₂-C₆-alkynyl, phenyl, phenyl-C₁-C₆-alkyl,
where the alkyl, alkenyl or alkynyl radicals mentioned may be partially or fully halogenated and/or substituted by one or more of the following radicals:
hydroxyl, mercapto, amino, cyano, nitro, formyl, C₁-C₄-alkylamino, C₁-C₄-dialkylamino, C₁-C₄-alkoxycarbonyl, C₁-C₄-alkylcarbonyl, C₁-C₄-alkylcarbonyloxy, C₁-C₄-alkyl, C₁-C₄-haloalkyl, C₁-C₄-alkylthio, C₁-C₄-haloalkylthio, C₁-C₄-alkoxy, C₁-C₄-haloalkoxy;
Het is a three- to six-membered, partially or fully saturated heterocyclic group having up to three heteroatoms selected from the group consisting of:
nitrogen, oxygen and sulfur,
where the heterocyclic group mentioned may be partially or fully halogenated and/or substituted by R⁵;
R⁵ is hydrogen, hydroxyl, mercapto, amino, cyano, nitro, formyl, C₁-C₄-alkylamino, C₁-C₄-dialkylamino, C₁-C₄-alkoxycarbonyl, C₁-C₄-alkylcarbonyl, C₁-C₄-alkylcarbonyloxy, C₁-C₄-alkyl, C₁-C₄-haloalkyl, C₁-C₄-alkylthio, C₁-C₄-haloalkylthio, C₁-C₄-alkoxy, C₁-C₄-haloalkoxy, where in all instances each of the alkyl radicals may be substituted by one or more of the following radicals:
cyano, formyl, C₁-C₄-alkylamino, C₁-C₄-dialkylamino, C₁-C₄-alkoxycarbonyl, C₁-C₄-alkylcarbonyl, C₁-C₄-alkylcarbonyloxy, C₁-C₄-alkyl, C₁-C₄-haloalkyl, C₁-C₄-alkylthio, C₁-C₄-halo-
alkylthio, C₁-C₄-alkoxy, C₁-C₄-haloalkoxy;
and agriculturally useful salts thereof.

2. The 2-benzoylcyclohexan-1,3-dione of the formula I according to claim 1, in which Het is a five- or six-membered, partially or fully saturated heterocyclic group having up to three heteroatoms selected from the group consisting of
nitrogen, oxygen and sulfur.

3. A process for preparing 2-benzoylcyclohexan-1,3-diones of the formula I according to claim 1 or 2, which comprises acylating an optionally substituted cyclohexane-1,3-dione Q with an activated carboxylic acid IIIa or with a carboxylic acid IIIb, 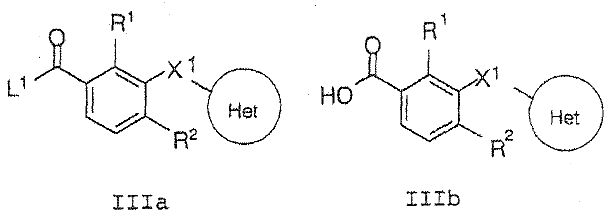 where the substituents R¹, R², X¹ and Het are each as defined in claim 1 and L¹ is a nucleophilically replaceable leaving group, and rearranging the acylation product, if appropriate in the presence of a catalyst, to the compounds I.

4. An activated carboxylic acid of the formula IIIa or a carboxylic acid of the formula IIIb according to claim 3 where the substituents R¹, R², X¹ and Het are each as defined in claim 1 and L¹ is a nucleophilically replaceable leaving group.

5. A composition comprising a herbicidally effective amount of at least one 2-benzoylcyclohexane-1,3-dione of the formula I or an agriculturally useful salt of I according to claim 1 or 2 and auxiliaries customary for the formulation of crop protection agents.

6. A process for preparing herbicidally effective compositions according to claim 5, which comprises mixing a herbicidally effective amount of at least one 2-benzoylcyclohexane-1,3-dione of the formula I or an agriculturally useful salt of I according to claim 1 or 2 and auxiliaries customary for the formulation of crop protection agents.

7. A process for controlling undesirable vegetation, which comprises allowing a herbicidally effective amount of at least one 2-benzoylcyclohexane-1,3-dione of the formula I or an agriculturally useful salt of I according to claim 1 or 2 to act on plants, their habitat and/or on seeds.

8. The use of the 2-benzoylcyclohexane-1,3-diones of the formula I and agriculturally useful salts thereof according to claim 1 or 2 as herbicides.

## Revendications

1. 2-Benzoylcyclohexane-1,3-diones de formule I 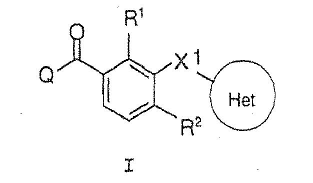 dans laquelle les substituants ont la signification suivante :
R¹ désigne nitro, halogène, cyano, rhodano, C₁-C₆-alkyle, C₁-C₆-halogénoalkyle, C₁-C₆-alcoxy-C₁-C₆-alkyle, C₂-C₆-alcényle, C₂-C₆-alcinyle, -OR³, ou -S(O)ₙR³ ;
R² représente hydrogène ou un radical tel que cité précédemment sous R¹ ;
R³ hydrogène, C₁-C₆-alkyle, C₁-C₆-halogénoalkyle, C₂-C₆-alcényle, C₂-C₆-alcinyle, phényle ou phényl-C₁-C₆-alkyle, lesdits radicaux alkyle pouvant être partiellement ou complètement halogénés et/ou pouvant porter un à trois des groupes suivants :
hydroxy, mercapto, amino, cyano, R³, -OR³,-SR³, -N(R³)₂, =NOR³, -OCOR³, -SCOR³, -NR³COR³,-CO₂R³, -COSR³, -CON(R³)₂, C₁-C₄-alkyliminooxy, C₁-C₄-alcoxyamino, C₁-C₄-alkylcarbonyle, C₁-C₄-alcoxy- C₂-C₆-alcoxyoarbonyle, C₁-C₄-alkylsulfonyle, hétérocyclyle, hétérocyclyloxy, phényle, benzyle, hétaryle, phénoxy, benzyloxy et hétaryloxy, les huit derniers radicaux pouvant, pour leur part, être substitués par un à trois atomes d'halogène et/ou un ou deux radicaux du groupe suivant : nitro, cyano, méthyle, trifluorométhyle, méthoxy, trifluorométhoxy ou méthoxycarbonyle ;
n 0, 1, ou 2;
Q un cycle cyclohexane-1,3-dione lié en position 2 de formule II 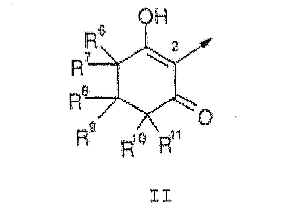
dans laquelle R⁶, R⁷, R⁹ et R¹¹ désignent hydrogène ou C₁-C₄-alkyle ;
R⁸ désigne hydrogène, C₁-C₄-alkyle ou C₁-C₄-cycloalkyle, les deux derniers groupes pouvant porter un à trois des substituants suivants :
halogène, C₁-C₄-alkylthio ou C₁-C₄-alcoxy;
ou
tétrahydropyran-2-yle, tétrahydropyran-3-yle, tétrahydropyran-4-yle, tétrahydrothiopyran-2-yle, tétrahydrothiopyran-3-yle, tétrahydrothiopyran-4-yle, 1,3-dioxolan-2-yle, 1,3-dioxan-2-yle, 1,3-oxathiolan-2-yle, 1,3-oxathian-2-yle, 1,3-dithiolan-2-yle ou 1,3-dithian-2-yle, les 6 derniers radicaux pouvant être substitués par un à trois radicaux C₁-C₄-alkyle ;
R¹⁰ désigne hydrogène, C₁-C₄-alkyle ou C₁-C₆-alcoxycarbonyle ;
ou
R⁸ et R¹¹ forment ensemble une liaison p ou un cycle carbocyclique tri- à hexavalent ;
ou
l'unité CR⁸R⁹ peut être remplacée par C=O ;
X¹ une chaîne C₁-C₂-alkylène- ou C₂-alcinylène ;
R⁴ hydrogène, C₁-C₆-alkyle, C₂-C₆-alcényle, C₂-C₆-alcinyle, phényle, phényl-C₁-C₆-alkyle, lesdits radicaux alkyl-, alcényle, alcinyle pouvant être partiellement ou complètement halogénés et/ou substitués par un ou plusieurs des radicaux suivants :
hydroxy, mercapto, amino, cyano, nitro, formyle, C₁-C₄-alkylamino, C₁-C₄-dialkylamino, C₁-C₄-alcoxycarbonyle, C₁-C₄-alkylcarbonyle, C₁-C₄-alkylcarbonyloxy, C₁-C₄-alkyle, C₁-C₄-halogénoalkyle, C₁-C₄-alkylthio, C₁-C₄-halogénoalkylthio, C₁-C₄-alcoxy, C₁-C₄-halogénoalcoxy ;
Het un groupe hétérocyclique tri- à hexavalent, saturé en tout ou en partie avec, au maximum, trois hétéroatomes sélectionnés dans le groupe :
azote, oxygène ou soufre,
ledit groupe hétérocyclique pouvant être partiellement ou complètement halogéné et/ou substitué par R⁵;
R⁵ hydrogène, hydroxy, mercapto, amino, cyano, nitro, formyle, C₁-C₄-alkylamino, C₁-C₄-dialkylamino, C₁-C₄-alcoxycarbonyle, C₁-C₄-alkylcarbonyle, C₁-C₄-alkylcarbonyloxy, C₁-C₄-alkyle, C₁-C₄-halogénoalkyle, C₁-C₄-alkylthio, C₁-C₄-halogénoalkylthio, C₁-C₄-alcoxy, C₁-C₄-halogénoalcoxy; les radicaux alkyle pouvant dans tous les cas être respectivement substitués par un ou plusieurs des radicaux suivants :
cyano, formyle, C₁-C₄-alkylamino, C₁-C₄-dialkylamino, C₁-C₄-alcoxycarbonyle, C₁-C₄-alkylcarbonyle, C₁-C₄-alkylcarbonyloxy, C₁-C₄-alkyle, C₁-C₄-halogénoalkyle, C₁-C₄-alkylthio C₁-C₄-halogénoalkylthio, C₁-C₄-alcoxy, C₁-C₄-halogénoalcoxy;
ainsi que leurs sels utilisables en agriculture.

2. 2-Benzoylcyclohexane-1,3-diones de formule I selon la revendication 1, dans lesquelles Het désigne un groupe hétérocyclique penta- à hexavalent partiellement ou totalement saturé avec au maximum trois hétéroatomes sélectionnés dans le groupe de l'azote, de l'oxygène ou du soufre.

3. Procédé de préparation de 2-benzoylcyclohexane-1,3-diones de formule I selon l'une des revendications 1 à 2, **caractérisé en ce que** l'on acyle une cyclohexane-1,3-dione Q éventuellement substituée avec un acide carboxylique IIIa activé ou avec un acide carboxylique IIIb 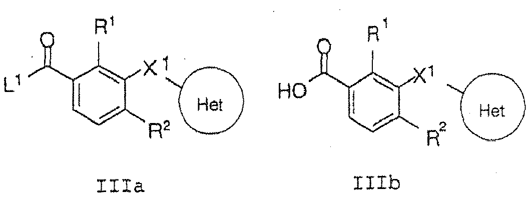 les substituants R¹, R², X¹ et Het ayant la signification spécifiée dans la revendication 1 et L¹ désignant un groupe partant échangeable nucléophilement et le produit d'acylation est superposé aux composés I, éventuellement en présence d'un catalyseur.

4. Acides carboxyliques activés de formule IIIa et acides carboxyliques de formule IIIb selon la revendication 3, les substituants R¹, R², X¹ et Het ayant la signification spécifiée dans la revendication 1 et L¹ désignant un groupe partant échangeable nucléophilement.

5. Produits contenant une quantité à action herbicide d'au moins une 2-benzoylcyclohexane-1,3-dione de formule I ou d'un sel utilisable en agriculture de I selon les revendications 1 à 2 et des adjuvants courants pour la formulation d'agents phytoprotecteurs.

6. Procédé de préparation de produits à action herbicide selon la revendication 5, **caractérisé en ce que** l'on mélange une quantité à action herbicide d'au moins une 2-benzoylcyclohexane-1,3-dione de formule I ou d'un sel utilisable en agriculture de I selon les revendications 1 à 2 et des adjuvants courants pour la formulation d'agents phytoprotecteurs.

7. Procédé de lutte contre la croissance de plantes indésirées, **caractérisé en ce que** l'on fait agir une quantité à action herbicide d'au moins une 2-benzoylcyclohexane-1,3-dione de formule I ou un sel utilisable en agriculture de I selon les revendications 1 à 2 sur des plantes, leur espace de vie et/ou sur des semences.

8. Utilisation de 2-benzoylcyclohexane-1,3-diones de formule I et de leurs sels utilisables en agriculture selon les revendications 1 à 2 comme herbicides.
